# EUROPEAN PATENT APPLICATION

(11) **EP 2 915 528 A1**
(43) Date of publication of application: **09.09.2015**
(21) Application number: 15157825.9
(22) Date of filing: 05.03.2015
(51) Int. Cl.: A61K 9/20, A61K 31/40

(54) **Vildagliptin formulation process under inert gas atmosphere**

(30) Priority: 06.03.2014 TR 201402685; 03.11.2014 TR 201412836
(71) Applicant: Sanovel Ilac Sanayi ve Ticaret A.S., 34460 Istanbul (TR)
(72) Inventor: Türkyilmaz, Ali, 34460 Istanbul (TR); Yelken, Gülay, 34460 Istanbul (TR); Saydam, Mehtap, 34460 Istanbul (TR); Ülgen, Onur, 34460 Istanbul (TR)
(74) Representative: Sevinç, Erkan

(57) **Abstract**

The present invention relates to a process for preparing a pharmaceutical composition of vildagliptin under oxygen free inert gas atmosphere. The present invention particularly relates to a pharmaceutical composition of vildagliptin obtained by the process which is stable to oxygen physiologically and chemically.

## Description

### Field of Invention

The present invention relates to a process for preparing a pharmaceutical composition of vildagliptin under oxygen free inert gas atmosphere. The present invention particularly relates to a pharmaceutical composition of vildagliptin obtained by the process which is stable to oxygen physiologically and chemically.

### Background of Invention

Vildagliptin is used for type 2 or non-insulin dependent diabetes. It increases the amount of insulin produced by the body. It also decreases the amount of glucagon which is produced by the body. Because of these effects, vildagliptin can help to control blood sugar levels in people with diabetes. Vildagliptin is used in combination with other medicines which help to control blood sugar levels.

DPP-IV inhibitors work by blocking the action of DPP-IV, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Vildagliptin works by binding to DPP-IV and preventing it from breaking down the GLP-1 and GIP. This increases the levels of these hormones in the body and so increases their effect on controlling blood sugar.

Its chemical name is (S)-{[(3-hydroxyadamantan-1-yl)amino]acetyl}pyrrolidine-2-carbonitril and its chemical structure is shown in the Formula I.

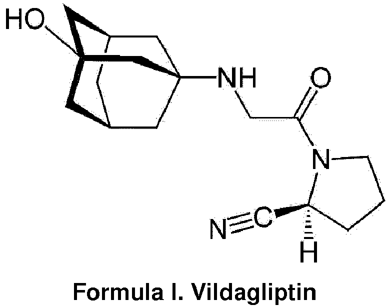

Vildagliptin is soluble in water and in organic polar solvents.

Vildagliptin is marketed under the trademark Galvus^{®} in 50 mg dosage forms. It is used against diabetes mellitus, but particularly in treating type 2 diabetes.

There are various patents in the patent literature in relation to vildagliptin.

The patent application WO0034241 discloses vildagliptin or an acid addition salt thereof, as well as its use in diabetes mellitus and obesity.

The patent application WO2006078593 claims a direct-compression formulation of a DPP-IV inhibitor compound, and preferably of vildagliptin or an acid addition salt thereof.

The patent application WO2006135723 discloses a formulation, comprising vildagliptin as an active agent, as well as hydroxypropyl methyl cellulose, microcrystalline cellulose, and magnesium stearate.

In the patent applications EP1841413A2 and EP2165703A3, direct compression is used to develop tablet formulation of DPP-IV inhibitor compounds, especially vildagliptin or an acid addition salt thereof.

Many conventional formulations of vildagliptin are disclosed in the patents referred to above.

On the other hand, stability problems are frequently encountered in vildagliptin formulations under the influence of ambiance and physical conditions. Vildagliptin is an active agent that is highly-susceptible to oxygen in the air and humidity conditions. When vildagliptin is initially exposed to oxygen in the air and humidity, it degrades structurally and develops chemical behavioral changes and it may lead to process related problems, stability problems during manufacturing, packaging and transportation. As a result of this, the main problem is that the stability of vildagliptin during the product development process is not at a desired level and in addition the shelf life of the product thereof is shortened. This fact causes potency loses and impurities occure in the formulation.

Above all, vildagliptin is unstable to oxygen physiologically and chemically. This novel invention particularly relates to a process for preparing a pharmaceutical composition of vildagliptin under inert gas from the beginning of the process until the end. The process comprises storing vildagliptin, preparing formulation, packaging, handling and storing finished dosage forms of vildagliptin. During the whole process oxygen and vildagliptin never come together so process related stability problems are overcomed.

### Detailed Description of Invention

The present invention provides a novel process for preparing a pharmaceutical composition of vildagliptin which comprises said process being under oxygen free inert gas atmosphere.

Accordingly, the main object of the present invention is to obtain a stable pharmaceutical composition of vildagliptin which is stable to oxygen physiologically and chemically.

In this present invention, the process comprises all the steps of storing vildagliptin, all manufacturing steps of drug product, packaging, handling and storing the finished dosage forms of vildagliptin.

In a preferred embodiment according to the present invention, said novelty is carried out with using inert gas during the whole process instead of oxygen. As a result, oxygen and vildagliptin never come together so process related stability problems are overcomed.

In a preferred embodiment according to the present invention, the inert gas is selected from the group comprising nitrogen, helium, neon, argon, krypton, xenon.

In another preferred embodiment according to the present invention, the inert gas is preferably nitrogen.

In a preferred embodiment according to the present invention, nitrogen atmosphere comprises nitrogen at least 99.9%.

In another preferred embodiment according to the present invention, nitrogen atmosphere comprises oxygen content which does not exceed 5 vpm (volume per millions).

According to the embodiment, nitrogen atmosphere comprises a humidity which does not exceed 3 vpm (volume per millions).

Another object of the present invention is to have a pharmaceutical composition of vildagliptin obtained by the process, wherein the pharmaceutical form of said vildagliptin composition is solid, semi-solid or liquid.

In a preferred embodiment according to the present invention, the pharmaceutical form of said vildagliptin composition is preferably solid dosage form for oral use.

The pharmaceutical composition as solid dosage form is selected from the group comprises tablets including compressed tablets, coated or uncoated tablets, bilayer tablets, multilayer tablets, buccal tablets, sublingual tablets, tablet in tablets, in-lay tablets, effervescent compositions, effervescent tablets, immediate release tablets, modified release tablets, ODT-ER (orally disintegrating tablets-extended release), film-coated tablets, orally disintegrating tablets, gastric disintegrating tablets, mini tablets; pills, capsules, hard or soft gelatin capsules, powders, pellets, coated bead systems, granules, microspheres, ion exchange resin systems, dragees, sachets; orally administrable thin films, solutions or solids.

In a preferred embodiment, the solid dosage form is preferably tablet or capsule.

In another preferred embodiment according to the present invention, said pharmaceutical formulation obtained by the process comprises;
a. 14.0 - 16.0 % by weight of vildagliptin
b. 3.0 - 25.0 % by weight of croscarmellose sodium
c. 0.1 - 3.0 % by weight of colloidal silicon dioxide
d. 0.1 - 25.0 % by weight of polyvinylpyrollidone
e. 70.0 - 80.0 % by weight of dibasic calcium phosphate
f. 0.01 - 5.0 % by weight of magnesium stearate
g. Optionally, coating

In a further preferred embodiment according to the present invention, said formulation is in the form of a tablet.

Another preferred embodiment according to the present invention provides said pharmaceutical formulation obtained by the process comprises the following process steps of;
a. mixing vildagliptin with colloidal silicon dioxide and sieving;
b. blending this mixture with croscarmellose sodium, dibasic calcium phosphate and polyvinylpyrollidone;
c. compaction and sieving;
d. adding magnesium stearate into the mixture obtained above and mixing the resulting mixture;
e. compressing the resulting mixture into tablets.

The whole process for the preparation of vildagliptin (coated or uncoated) tablet formulation occurs under inert gas atmosphere.

In another embodiment according to the present invention, the said dosage form is stored in a blister package.

The blister package is selected from the group comprises aluminium, polyvinyl chloride (PVC), polychlorotrifluoro ethylene (PCTFE), cyclic olefin copolymers (COC), cyclic olefin polymers (COP), polypropylene (PP), polyethylene (PE), glycol-modified polyethylene terephthalate (PETg), high impact polystyrene (HIPS), polystyrene, crystalline polyethylene terephthalate (CPET) or mixtures thereof.

According to the present invention, the pharmaceutical composition is used for preventing or treating the diabetes disease in mammalians and particularly in humans.

### Example: Vildagliptin tablet formulation process under nitrogen atmosphere

| **Ingredients** | **Amount (%)** |
|---|---|
| vildagliptin | 15.6 |
| croscarmellose sodium | 5.0 |
| colloidal silicon dioxide | 0.6 |
| dibasic calcium phosphate | 74.5 |
| polyvinylpyrollidone | 3.1 |
| magnesium stearate | 1.1 |
| coating (optionally) | 0.2 - 5.0 |

**Production process of the formulation:** The whole process for the preparation of vildagliptin (coated or uncoated) tablet formulation occurs under nitrogen atmosphere. The production of the formulation is carried out as follows: Vildagliptin, colloidal silicone dioxide (Aerosil 200) are sieved and mixed. Croscarmellose sodium, polyvinylpyrollidone (PVP 25) and dibasic calcium phosphate are added to the mixture (Ac-Di-Sol) and mixed. The mixture is compacted in compactor and sieved. Magnesium stearate is sieved and added into obtained dry granules and mixed. Final mixture is pressed into tablets. Optionally, tablets are coated for moisture protection.

## Claims

1. A process for preparing a pharmaceutical composition of vildagliptin comprising said process being under oxygen free inert gas atmosphere.

2. The process according to claim 1, wherein the inert gas is selected from the group comprising nitrogen, helium, neon, argon, krypton, xenon.

3. The process according to claim 2, wherein the inert gas is preferably nitrogen.

4. The process according to claim 3, wherein nitrogen atmosphere comprising nitrogen at least 99.9%.

5. The process according to claim 4, wherein nitrogen atmosphere comprising oxygen content which does not exceed 5 vpm (volume per millions).

6. The process according to claim 4, wherein nitrogen atmosphere comprising humidity which does not exceed 3 vpm (volume per millions).

7. A pharmaceutical composition of vildagliptin obtained by process according to any preceding claims, wherein the pharmaceutical form of said vildagliptin composition is solid, semi-solid or liquid.

8. The pharmaceutical composition according to claim 7, wherein the pharmaceutical form of said vildagliptin composition is preferably solid dosage form for oral use.

9. The pharmaceutical composition according to claim 8, wherein the solid dosage form is selected from the group comprising tablets including compressed tablets, coated or uncoated tablets, bilayer tablets, multilayer tablets, buccal tablets, sublingual tablets, tablet in tablets, in-lay tablets, effervescent compositions, effervescent tablets, immediate release tablets, modified release tablets, ODT-ER (orally disintegrating tablets-extended release), film-coated tablets, orally disintegrating tablets, gastric disintegrating tablets, mini tablets; pills, capsules, hard or soft gelatin capsules, powders, pellets, coated bead systems, granules, microspheres, ion exchange resin systems, dragees, sachets; orally administrable thin films, solutions or solids.

10. The pharmaceutical composition according to claim 9, wherein solid dosage form is preferably tablet or capsule.

11. The pharmaceutical composition according to claims 7 to 10, comprising;
a. 14.0 - 16.0 % by weight of vildagliptin
b. 3.0 - 25.0 % by weight of croscarmellose sodium
c. 0.1 - 3.0 % by weight of colloidal silicon dioxide
d. 0.1 - 25.0 % by weight of polyvinylpyrollidone
e. 70.0 - 80.0 % by weight of dibasic calcium phosphate
f. 0.01 - 5.0 % by weight of magnesium stearate
g. Optionally, coating

12. The pharmaceutical composition according to claim 11, comprising the process steps of;
a. mixing vildagliptin with colloidal silicon dioxide and sieving;
b. blending this mixture with croscarmellose sodium, dibasic calcium phosphate and polyvinylpyrollidone;
c. compaction and sieving;
d. adding magnesium stearate into the mixture obtained above and mixing the resulting mixture;
e. compressing the resulting mixture into tablets.

13. The pharmaceutical composition according to any of the preceding claims, wherein the said dosage form is stored in a blister package.

14. The pharmaceutical composition according to claim 13, wherein said blister package comprising aluminium, polyvinyl chloride (PVC), polychlorotrifluoro ethylene (PCTFE), cyclic olefin copolymers (COC), cyclic olefin polymers (COP), polypropylene (PP), polyethylene (PE), glycol-modified polyethylene terephthalate (PETg), high impact polystyrene (HIPS), polystyrene, crystalline polyethylene terephthalate (CPET) or mixtures thereof.

15. The pharmaceutical composition according to any of the preceding claims, for use in preventing or treating the diabetes disease in mammalians and particularly in humans.
